# EUROPEAN PATENT SPECIFICATION

(11) **EP 4 001 885 B1**
(45) Date of publication and mention of the grant of the patent: **19.06.2024**
(21) Application number: 21208619.3
(22) Date of filing: 16.11.2021
(51) Int. Cl.: G01N 1/22, B01L 7/00, B64D 11/04, B64D 11/00

(54) **SYSTEMS AND METHODS TO OBTAIN A BIOLOGICAL SAMPLE REPRESENTATIVE OF A PASSENGER CABIN ON AN AIRCRAFT AUTOMATICALLY FROM THE COLLECTOR DEVICE**
SYSTEME UND VERFAHREN ZUR AUTOMATISCHEN ENTNAHME AUS EINER SAMMELVORRICHTUNG EINER FÜR DIE PASSAGIERKABINE REPRÄSENTATIVEN BIOLOGISCHEN PROBE IN EINEM FLUGZEUG
SYSTÈMES ET PROCÉDÉS POUR OBTENIR UN ÉCHANTILLON BIOLOGIQUE REPRÉSENTATIF DE LA CABINE DE PASSAGERS D'UN AÉRONEF AUTOMATIQUEMENT À PARTIR DU DISPOSITIF DE COLLECTE

(30) Priority: 16.11.2020 US 202063114386 P; 16.11.2020 US 202063114330 P; 16.11.2020 US 202063114339 P; 16.11.2020 US 202063114400 P; 16.11.2020 US 202063114064 P; 16.11.2020 US 202063114350 P; 16.11.2020 US 202063114157 P; 16.11.2020 US 202063114366 P
(43) Date of publication of application: 25.05.2022
(73) Proprietor: B/E Aerospace, Inc., Winston-Salem, NC 27105 (US)
(72) Inventor: GONZALEZ, Arnau Gastillo, 3607 AK Maarseen (NL); SURAWSKI, Eric, Hebron, CT 06248 (US); GONZALEZ, Vanessa, CP 07004, Palma de Mallorca (Baleares) (ES); MURCIA, Antonio Martinez, CP 03317, Orihuela (Alicante) (ES)
(74) Representative: Dehns

(56) References cited:
- US-A1- 2004 038 385
- US-A1- 2011 159 596
- US-A1- 2014 370 519
- US-B1- 6 468 330
- US-B1- 7 390 339
- KORVES T. M. ET AL: "Bacterial communities in commercial aircraft high-efficiency particulate air (HEPA) filters assessed by PhyloChip analysis :", INDOOR AIR, vol. 23, no. 1, 8 June 2012 (2012-06-08), pages 50-61, XP055907740, DK ISSN: 0905-6947, DOI: 10.1111/j.1600-0668.2012.00787.x

## Description

### Background

### Priority Claim

The following application claims priority to U.S. Provisional Patent Applications with the following Serial Nos. 63/114,330, 63/114,339, 63/114,350, 63/114,400, 63,114,064, 63/114,157, 63/114,386, 63/114,366 all filed on November 16, 2020; and Patent Application Serial No. 63/043,414 filed on June 24, 2020.

### Technological Field

The present application is related to a system and method used collect a representative air sample of an aircraft, more specifically to a method and systems for collecting a biological sample on an aircraft using an automated collector.

### Description of Related Art

The spread progression of SARS-CoV-2 around the world has risen a red flag: Economic economic globalization creates systemic risks. As trade, finance, travel, cyber and other networks grow in scale and interact, they become more complex and unstable. The transporters of the goods of the global economy, such as major airport hubs, are also spreaders of the pathogens. The 2008 global financial crisis provided a dramatic example of how contagions could spread from the US to global markets overnight. So too has the rapid spread of cyber viruses. In health, rising life expectancy and success in preventing a repeat of the devastating influenza pandemic of 1918, which infected about one-third of the world's population and killed as many as 50m people, has created a false sense of security. But the world is now more interdependent. For example, China represents almost one-fifth of global output, is integral to global supply chains, and its tourists spend over $260 billion annually. The CovidCOVID-19CoVID-19 pandemic shed light on the need for better monitoring, detecting, and isolating ill passengers, specifically due to the havoc that was wreaked detrimental impact on the global economy, specifically air travel due to closed borders, movement restrictions, and testing requirements.

However, the COVIDCoVID-19 pandemic the air travel industry has proven that air travel can be safe and that aircraft cabins have a well-managed airflow that inhibits minimize the risk for transmission of virus, and that being seated onboard an aircraft is safer than shopping in large stores. Governments and other authorities need to assume that aircraft are contaminated until proven "clean", as 25% of COVID-19 cases are asymptomatic or pre-symptomatic; but still contagious. Thus, if borders shutdown and a drastic reduction in international travel global passenger travel is greatly reduced. To date travelers and governments have relied on individual diagnostic tests. The uncertainty of the results has reduced people's inclination to travel and subsequent airline inclination to maintain routes.

Accordingly, conventional systems and methods of monitoring infections has not lived up to requirements of the fast-paced modern world. Thus, there is still a need in the art for an improved on-board virus and pathogen detection system. The present disclosure provides a solution for this need. US 2004/038385 A1 describes a system for autonomous monitoring of bioagents. KORVES T. M. ET AL: "Bacterial communities in commercial aircraft high-efficiency particulate air (HEPA) filters assessed by PhyloChip analysis :", INDOOR AIR, vol. 23, no. 1, 8 June 2012 (2012-06-08), pages 50-61, ISSN: 0905-6947, DOI: 10.1111/j.1600-0668.2012.00787.x, disclose the use of HEPA filter samples and the G2 PhyloChip to analyze microbial communities in aircraft air and their comparison to bacterial communities in ambient outdoor air from North American cities.

### Summary of the Invention

A system for monitoring and collecting pathogens from aircraft air is disclosed. The system includes a collector for collecting particulate samples positioned within at least one of an outlet flow path or a recirculation flow path, at least one of an outflow valve positioned in the outlet flow path downstream from the collector, and a retrieval device for retrieving the collector from the outflow valve. The particulate samples can include droplets exhaled from passengers throughout a duration of a flight. The collector can include a filter material within the collector. The outlet flow path can be a main cabin outlet flow path and wherein the outflow valve is positioned within the outlet flow path, and where the collector is positioned upstream from the outflow valve to collect a cabin air particulate sample throughout the duration of a flight.

The retrieval device can be motorized, and include an arm for extending into the outflow valve to retrieve the collector. The retrieval device can be configured to be wirelessly controlled by a user remote to the retrieval device or to be driven and operated by a user onsite at the retrieval device.

A method for collecting particulates from aircraft air using the system described above includes capturing particulates in at least one of an outlet flow path or a recirculation flow path with a collector for a period of time, retrieving the collector from at least one of the outlet flow path or the recirculation flow path for testing by a user located at a location remote to the aircraft, and inserting a clean collector into at least one of the outlet flow path or the recirculation flow path for use during another period of time. The method can include a motorized vehicle to do the retrieval. The collector can be dispatched to a remote location to do the PCR testing or a PCR test can be done onboard the motorized vehicle. A result of the PCR test can be relayed to a central data center.

These and other features of the systems and methods of the subject disclosure will become more readily apparent to those skilled in the art from the following detailed description of the preferred embodiments taken in conjunction with the drawings.

### Brief Description of the Drawings

So that those skilled in the art to which the subject disclosure appertains will readily understand how to make and use the devices and methods of the subject disclosure without undue experimentation, embodiments thereof will be described in detail herein below with reference to the figure, wherein:

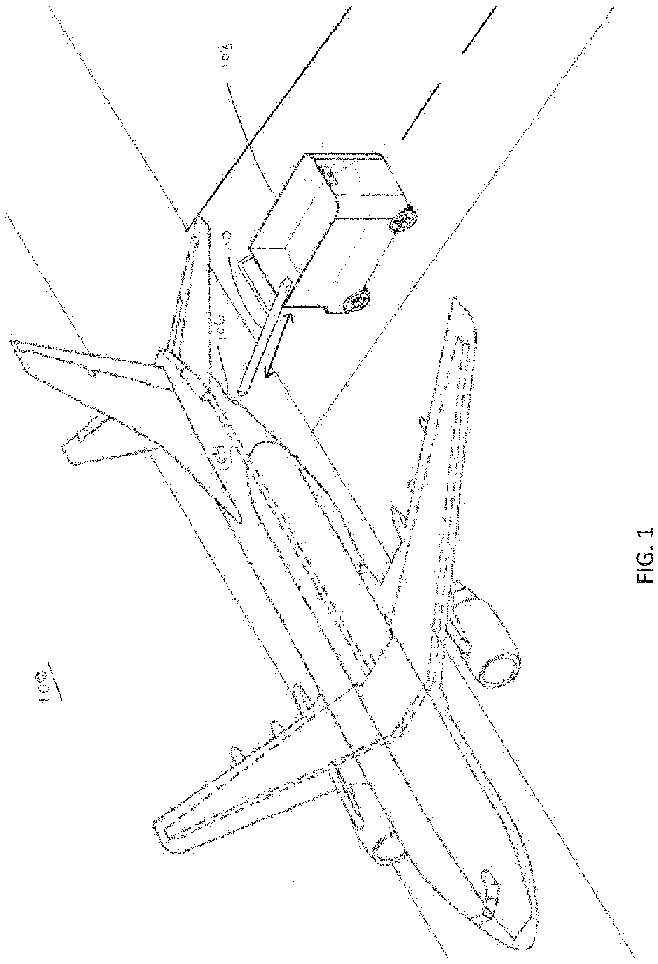

FIG. 1 is a block diagram of the system for collecting an sample from the aircraft in accordance with the disclosure.

### Detailed Description

Reference will now be made to the drawings wherein like reference numerals identify similar structural features or aspects of the subject disclosure. For purposes of explanation and illustration, and not limitation, a partial view of an exemplary embodiment of a system in accordance with the disclosure is shown in FIG. 1 and is designated generally by reference character 100. The global virus detection system described below is used to collect a bulk sample, representative of each passenger on the aircraft and to test it to provide a bulk screening of the aircraft upon arrival of the aircraft at a destination.

FIG. 1 shows a system 100 for retrieving a collected sample from an aircraft. The system 100 includes a vehicle 108 for collecting particulate samples from a collector positioned within at least one of an outlet flow path 104 or a recirculation flow path, at least one of an outflow valve 106 positioned in the outlet flow path downstream from the collector. The particulate samples that are collected can include droplets exhaled from passengers throughout a duration of a flight. The retrieval device 108 can be motorized, and include an arm 110 for extending into the outflow valve 106 to retrieve the collector. The arm 110 is shown extended into the outflow valve 106. The retrieval device 108 can be configured to be wirelessly controlled by a user remote to the retrieval device or to be driven and operated by a user onsite at or within the retrieval device 108.

A method for collecting particulates from aircraft air using the system described above includes capturing particulates in at least one of an outlet flow path or a recirculation flow path with a collector for a period of time, retrieving the collector from at least one of the outlet flow path or the recirculation flow path for testing by a user located at a location remote to the aircraft, and inserting a clean collector into at least one of the outlet flow path or the recirculation flow path for use during another period of time. The method can include a motorized vehicle to do the retrieval. The collector can be dispatched to a remote location to do the PCR testing or a PCR test can be done onboard the motorized vehicle. A result of the PCR test can be relayed to a central data center.

The methods and systems of the present disclosure, as described above and shown in the drawings, provide for an improved bulk data and analysis of passenger pathogens on an aircraft. The system provides a more efficient primary health controls at airports, helps identify at early stages surging pathogens and origin, and empower their scientific research (either academic and/or commercial / institutional).

## Claims

1. A system (100) for monitoring aircraft air comprising:
a collector for collecting particulate samples positioned within at least one of an outlet flow path or a recirculation flow path; and **characterized by**
at least one of an outflow valve (106) positioned in the outlet flow path downstream from the collector; and
a retrieval device (108) for retrieving the collector from the outflow valve.

2. The system of claim 1, wherein the collector includes a filter material

3. The system of any preceding claim, wherein the retrieval device (108) is motorized.

4. The system of any preceding claim, wherein the retrieval device (108) includes an arm for extending into the outflow valve to retrieve the collector.

5. The system of claim 4, wherein the arm is extendable

6. The system of any preceding claim, wherein the collector includes an adaptor and a filter material operatively connected to the adaptor.

7. The system of any preceding claim, wherein the retrieval device is configured to be wirelessly controlled by a user remote to the retrieval device.

8. The system of any preceding claim, wherein the retrieval device is configured to be driven and operated by a user onsite at the retrieval device.

9. The system of any preceding claim, wherein the outlet flow path is a main cabin outlet flow path and wherein the outflow valve is positioned within the cabin outlet flow path; and wherein the collector is positioned across the general cabin outlet flow path upstream from the outflow valve to collect a cabin air particulate sample throughout a duration of a flight.

10. A method for collecting particulates from aircraft air using the system of claim 1 comprising:
capturing particulates in at least one of said outlet flow path or recirculation flow path with said collector for a period of time during flight;
retrieving the collector from at least one of the outlet flow path or the recirculation flow path for testing by a user located at a location remote to the aircraft; and
inserting a clean collector into at least one of the outlet flow path or the recirculation flow path for use during another period of time.

11. The method of claim 10, wherein the retrieval is done by a motorized vehicle.

12. The method of claim 11, further comprising dispatching the collector to a remote location for pathogen/contaminant diagnostic testing, such as PCR testing.

13. The method of claim 9, further comprising providing the collector to a pathogen/contaminant diagnostic tester, such as PCR test, onboard the motorized vehicle.

14. The method of claim 12, further comprising relaying a result of the pathogen/contaminant diagnostic tester to a central data center.

15. The method of claim 10, further comprising removing filter material from an adaptor in order to retrieve the collected sample.

## Patentansprüche

1. System (100) zur Überwachung von Flugzeugluft, umfassend:
einen Sammler zum Sammeln von Partikelproben, der in mindestens einem von einem Auslassströmungsverlauf oder einem Rückführungsströmungsverlauf angeordnet ist; und **gekennzeichnet durch**
mindestens eines von einem Auslassventil (106), das im dem Auslassströmungsverlauf stromabwärts von dem Sammler angeordnet ist; und
einer Rückholvorrichtung (108) zum Zurückholen des Sammlers aus dem Auslassventil.

2. System nach Anspruch 1, wobei der Sammler ein Filtermaterial beinhaltet.

3. System nach einem der vorhergehenden Ansprüche, wobei die Rückholvorrichtung (108) motorisiert ist.

4. System nach einem der vorhergehenden Ansprüche, wobei die Rückholvorrichtung (108) einen Arm beinhaltet, der in das Auslassventil hineinragt, um den Sammler zurückzuholen.

5. System nach Anspruch 4, wobei der Arm ausfahrbar ist.

6. System nach einem der vorhergehenden Ansprüche, wobei der Sammler einen Adapter und ein Filtermaterial beinhaltet, das funktional mit dem Adapter verbunden ist.

7. System nach einem der vorhergehenden Ansprüche, wobei die Rückholvorrichtung dazu ausgestaltet ist, durch einen von der Rückholvorrichtung entfernten Benutzer drahtlos gesteuert zu werden.

8. System nach einem der vorhergehenden Ansprüche, wobei die Rückholvorrichtung dazu ausgestaltet ist, durch einen Benutzer vor Ort an der Rückholvorrichtung gesteuert und bedient zu werden.

9. System nach einem der vorhergehenden Ansprüche, wobei der Auslassströmungsverlauf ein Hauptkabinenauslassströmungsverlauf ist und wobei das Auslassventil innerhalb des Kabinenauslassströmungsverlaufs positioniert ist; und wobei der Sammler über dem allgemeinen Kabinenauslassströmungsverlauf stromaufwärts von dem Auslassventil positioniert ist, um während der gesamten Dauer eines Fluges eine Kabinenluftpartikelprobe zu sammeln.

10. Verfahren zum Sammeln von Partikeln aus Flugzeugluft unter Verwendung des Systems von Anspruch 1, umfassend:
Auffangen von Partikeln in mindestens einem von dem Auslassströmungsverlauf oder dem Rückführungsströmungsverlauf mit dem Sammler für einen Zeitraum während des Fluges;
Zurückholen des Sammlers aus mindestens einem von dem Auslassströmungsverlauf oder dem Rückführungsströmungsverlauf zum Testen durch einen Benutzer, der sich an einem von dem Flugzeug entfernten Ort befindet; und
Einsetzen eines sauberen Sammlers in mindestens einen von dem Auslassströmungsverlauf oder dem Rückführungsströmungsverlauf zur Verwendung während eines anderen Zeitraums.

11. Verfahren nach Anspruch 10, wobei das Zurückholen durch ein motorisiertes Fahrzeug erfolgt.

12. Verfahren nach Anspruch 11, ferner umfassend Versenden des Sammlers an einen entfernten Standort für einen Krankheitserreger-/Verunreinigungstest, wie beispielsweise einen PCR-Test.

13. Verfahren nach Anspruch 9, ferner umfassend Bereitstellen des Sammlers für einen Krankheitserreger-/Verunreinigungstest, wie etwa einen PCR-Test, an Bord des motorisierten Fahrzeugs.

14. Verfahren nach Anspruch 12, ferner umfassend Weiterleiten eines Ergebnisses des Krankheitserreger-/Verunreinigungsdiagnosetests an ein zentrales Datenzentrum.

15. Verfahren nach Anspruch 10, ferner umfassend Entfernen von Filtermaterial von einem Adapter, um die gesammelte Probe zu zurückzuholen.

## Revendications

1. Système (100) de surveillance de l'air d'un aéronef comprenant :
un collecteur destiné à collecter des échantillons de particules positionné dans au moins l'un parmi un circuit d'écoulement de sortie ou un circuit d'écoulement de recirculation ; et **caractérisé par**
au moins l'un parmi une vanne de régulation échappement (106) positionnée dans le circuit d'écoulement de sortie en aval du collecteur ; et
un dispositif de récupération (108) destiné à récupérer le collecteur de la vanne de régulation échappement.

2. Système selon la revendication 1, dans lequel le collecteur comporte un matériau filtrant.

3. Système selon une quelconque revendication précédente, dans lequel le dispositif de récupération (108) est motorisé.

4. Système selon une quelconque revendication précédente, dans lequel le dispositif de récupération (108) comporte un bras destiné à s'étendre dans la vanne de régulation échappement pour récupérer le collecteur.

5. Système selon la revendication 4, dans lequel le bras est extensible.

6. Système selon une quelconque revendication précédente, dans lequel le collecteur comporte un adaptateur et un matériau filtrant connecté de manière fonctionnelle à l'adaptateur.

7. Système selon une quelconque revendication précédente, dans lequel le dispositif de récupération est configuré pour être commandé par le réseau sans fil par un utilisateur distant du dispositif de récupération.

8. Système selon une quelconque revendication précédente, dans lequel le dispositif de récupération est configuré pour être piloté et actionné par un utilisateur sur site au niveau du dispositif de récupération.

9. Système selon une quelconque revendication précédente, dans lequel le circuit d'écoulement de sortie est un circuit d'écoulement de sortie de cabine principal et dans lequel la vanne de régulation échappement est positionnée à l'intérieur du circuit d'écoulement de sortie de cabine ; et dans lequel le collecteur est positionné à travers le circuit d'écoulement de sortie de cabine général en amont de la vanne de régulation échappement pour collecter un échantillon de particules d'air de cabine pendant toute la durée d'un vol.

10. Procédé de collecte de particules de l'air d'un aéronef utilisant le système selon la revendication 1 comprenant :
la capture de particules dans au moins l'un parmi ledit circuit d'écoulement de sortie ou le circuit d'écoulement de recirculation avec ledit collecteur pendant une période pendant le vol ;
la récupération du collecteur depuis au moins l'un parmi le circuit d'écoulement de sortie ou le circuit d'écoulement de recirculation pour un test par un utilisateur situé à un emplacement éloigné de l'aéronef ; et
l'insertion d'un collecteur propre dans au moins l'un parmi le circuit d'écoulement de sortie ou le circuit d'écoulement de recirculation pour une utilisation pendant une autre période.

11. Procédé selon la revendication 10, dans lequel la récupération est effectuée par un véhicule motorisé.

12. Procédé selon la revendication 11, comprenant en outre l'envoi du collecteur vers un emplacement distant pour un test diagnostic d'agent pathogène/contaminant, tel qu'un test PCR.

13. Procédé selon la revendication 9, comprenant en outre la fourniture du collecteur à un testeur de diagnostic d'agent pathogène/contaminant, tel qu'un test PCR, à bord du véhicule motorisé.

14. Procédé selon la revendication 12, comprenant en outre le relais d'un résultat du testeur de diagnostic d'agent pathogène/contaminant à un centre de données central.

15. Procédé selon la revendication 10, comprenant en outre le retrait d'un matériau filtrant d'un adaptateur afin de récupérer l'échantillon collecté.
